# EUROPEAN PATENT SPECIFICATION

(11) **EP 4 297 842 B1**
(45) Date of publication and mention of the grant of the patent: **12.02.2025**
(21) Application number: 21712365.2
(22) Date of filing: 23.02.2021
(51) Int. Cl.: A61M 60/117, A61M 60/268, A61M 60/449, A61M 60/523, A61M 60/538, A61M 60/546, A61M 60/569, A61M 60/585, A61M 60/851, F04B 43/04, F04B 49/06

(54) **SYSTEM FOR DRIVING A PULSATILE FLUID PUMP**
SYSTEM ZUM ANTRIEB EINER PULSIERENDEN FLÜSSIGKEITSPUMPE
SYSTÈME D'ENTRAÎNEMENT D'UNE POMPE À FLUIDE PULSATILE

(43) Date of publication of application: 03.01.2024
(73) Proprietor: Ventriflo, Inc., Pelham, NH 03076 (US)
(72) Inventor: VINCENT, Douglas E., No. 7 Pelham, New Hampshire 03076 (US); BAILEY, Brian, No. 7 Pelham, New Hampshire 03076 (US); BZURA, Conrad, No. 7 Pelham, New Hampshire 03076 (US); OLNEY, David, No. 7 Pelham, New Hampshire 03076 (US); SMITH, Eric, No. 7 Pelham, New Hampshire 03076 (US); NABER, Jeffrey P., No. 7 Pelham, New Hampshire 03076 (US); LABONTÉ, Judy, No. 7 Pelham, New Hampshire 03076 (US); VINCENT, Kathleen, No. 7 Pelham, New Hampshire 03076 (US); MURPHY, Matthew J., No. 7 Pelham, New Hampshire 03076 (US); SHIELDS, Patrick, No. 7 Pelham, New Hampshire 03076 (US)
(74) Representative: Potter Clarkson
(86) International application number: PCT/US2021/019262
(87) International publication number: WO 2022/182334

(56) References cited:
- US-A1- 2012 245 405
- US-A1- 2015 078 934
- US-A1- 2020 289 731
- US-B2- 7 850 593

## Description

### Related Applications

The present application is one of four applications being filed on the same day and bearing attorney docket numbers 4747/1001, 4747/1002, 4747/1003, and 4747/1004.

### Technical Field

The present invention relates to pulsatile fluid pumps, and more particularly to pulsatile fluid pumps suitable for pumping blood.

### Background Art

A pulsatile fluid pump is taught in U.S. patent 7,850,593 ("our prior patent") for an invention of Douglas Vincent and Matthew Murphy, who are co-inventors of the present invention. Our prior patent discloses a pump actuated by a linear motor configured to cause reciprocation of a flexible membrane, serving as a wall of a fluid housing, that is in turn coupled to a pair of ball valves, in a manner as to implement pulsatile fluid flow. Further relevant prior art is for instance disclosed in documents US2020/289731 A1, US2012/245405 A1 and US2015/078934 A1.

### Summary of the Embodiments

A pulsatile fluid pump system according to the present invention comprises the technical features of independent claim 1.

The pulsatile fluid pump system for driving a fluid pump assembly includes a reciprocating linear motor having a magnet and a coil, the magnet moving in relation to the coil, the coil having an electrical input. The pulsatile fluid pump system further includes a controller system having an electrical output coupled to the electrical input of the coil, and the controller system is configured to execute a waveform program defining an electrical waveform at the electrical output. The waveform program is configured to control operation of the linear motor by modification of a feature, selected from the group consisting of amplitude, frequency, shape, and combinations thereof, of the electrical waveform at the electrical output. The waveform program is further configured to accept a set of user-specifiable parameters defining the performance of the linear motor and to modify the electrical waveform in response to such parameters.

Optionally, the pulsatile fluid pump system further includes a graphic display, coupled to the controller system, the controller system executing a graphics program configured to cause the graphic display to show a set of user-specifiable parameters defining the performance of the linear motor.

Optionally, the pulsatile fluid pump system further includes a flow sensor mechanically coupled to a fluid path including the integrated pump assembly, the flow sensor having an electrical output coupled to the controller system, wherein the controller system is executing a graphics program configured to cause the graphic display to show a set of items, including values of a set of user-specifiable parameters defining the performance of the linear motor and values of a set of physical flow characteristics.

Optionally, the set of items shown includes an instantaneous flow rate waveform in near real-time. Optionally, set of items shown includes an instantaneous stroke volume waveform in near real-time.

Optionally, the waveform program is configured to generate the electrical waveform at the electrical output by repeatedly performing a multi-piece polynomial spline algorithm in a manner responsive to a set of user-specifiable parameters defining the performance of the linear motor.

According to the invention as claimed, the controller system has a storage system in which is stored an archetype electrical waveform, and the waveform program reads the archetype electrical waveform from the storage system and modifies the archetype electrical waveform, based upon a set of user-specifiable parameters defining the performance of the linear motor, to generate the electrical waveform at the electrical output.

Optionally, the pulsatile fluid pump system further includes a set of sensors, electrically coupled to the controller system and configured to produce a set of sensor outputs corresponding to pumping performance. The waveform program is configured to generate the electrical waveform at the electrical output in a manner responsive to the set of sensor outputs and a set of user-specifiable parameters.

Also optionally, the user of the pulsatile fluid pump system may choose from a set of waveform programs. Optionally, the graphic display is touch sensitive.

### Brief Description of the Drawings

The foregoing features of embodiments will be more readily understood by reference to the following detailed description, taken with reference to the accompanying drawings, in which:
Fig. 1 is a vertical section of the pulsatile fluid pump system 301 showing the controller system **311,** power amplifier **321,** linear motor **330** (coil **332,** cooling fins **334,** and magnet **339),** push rod assembly **341,** flexible seal **351,** control housing **361,** and the integral pump assembly **200.**
Fig. **2** is an example of the touch-sensitive graphic display **400** user interface showing the user-specifiable motor parameters **401,** flow characteristics **411,** and user-specifiable input parameters **421.**
Fig. **3** is a vertical section of the push rod assembly **341.**
Fig. **4** is a vertical section of the linear motor **330.**
Fig. **5** is a block diagram describing a waveform program
Fig. **6** is a block diagram describing a first embodiment **511a** of the waveform program **511.**
Fig. **7** is a block diagram describing a second embodiment **511b** of the waveform program **511.**
Fig. **8** is a block diagram describing a graphics program **611.**

### Detailed Description of Specific Embodiments

Definitions. As used in this description and the accompanying claims, the following terms shall have the meanings indicated, unless the context otherwise requires:
A "set" includes at least one member.

An "electrical waveform" is a waveform selected from the group consisting of an electrical current waveform, a voltage waveform, and combinations thereof.

The term "user-specifiable input parameter" includes a user-definable attribute pertinent to an alarm setting or calculation for a user interface, such as low flow limit **421a**, high flow limit **421b,** and body surface area **421c** (BSA), as well as combinations of any of the foregoing attributes.

The term "user-specifiable parameter defining the performance of the linear motor" in the course of pumping includes a motor performance attribute such as stroke strength **401a,** beat rate **401b,** flow rate, average flow rate, stroke volume, flow index, pulse pressure, output pressure, magnet displacement, as well as combinations of any of the foregoing attributes.

The term "physical flow characteristic" includes a measured attribute such as stroke strength, beat rate, flow rate, average flow rate **411a**, stroke volume **411b,** flow index **411c**, pulse pressure, flow rate waveform **412,** stroke volume waveform **413,** duration over which the pump has been running (e.g., measured by timer **414),** as well as combinations of any of the foregoing attributes. If an attribute is user-specified in a given embodiment of the present invention, then measurement of the attribute is of subsidiary importance since its value has been specified. Similarly, if an attribute being measured has primary importance in a given embodiment of the present invention, then the parameter would not have been user-specified.

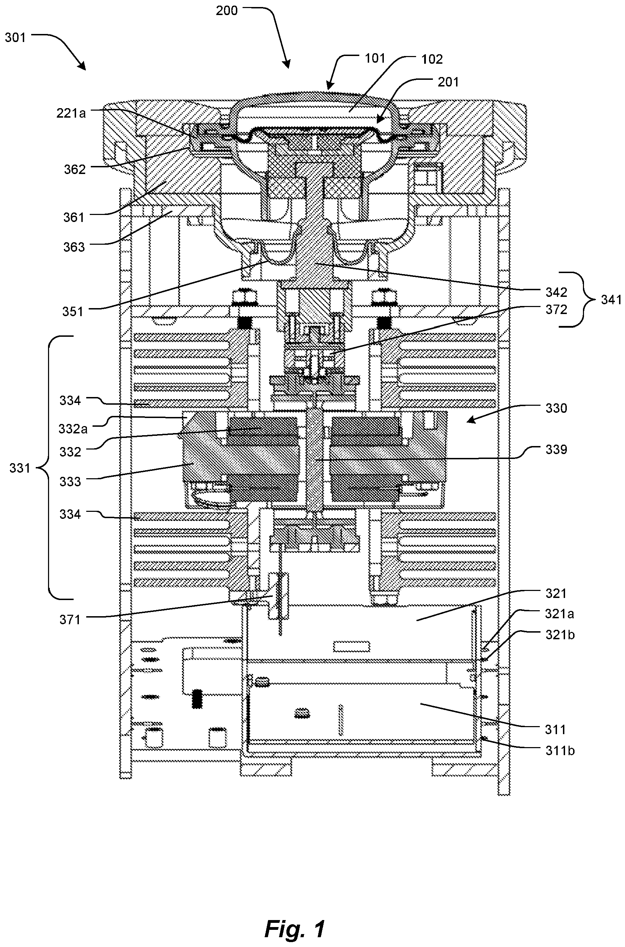

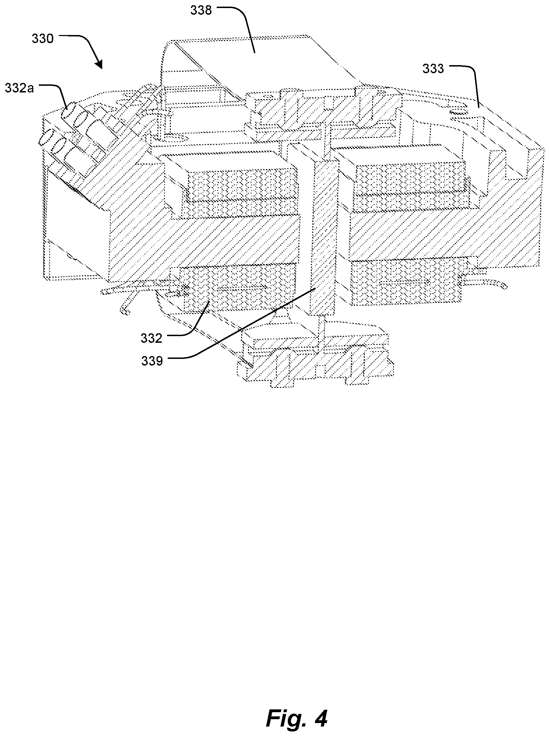

Fig. **1** is a vertical section of the pulsatile fluid pump system **301** showing the controller system **311** (with electrical output **311b),** power amplifier **321** (with electrical input **321a** and electrical output **321b),** linear motor **330** (comprised of a stationary member **331** which includes a coil **332** [with electrical input **332a],** a frame **333,** and cooling fins **334,** and a moving member which includes a spring **338** of Fig. **4** and a magnet **339),** position sensor **371,** push rod assembly **341** (comprised of a push rod **342** and force sensor **372),** flexible seal **351,** control housing **361,** and chassis **363.** An integral pump assembly **200** (comprised of the pump-valving assembly **101** with chamber **102,** diaphragm assembly **201,** and peripheral flange **221a**) is held by the peripheral flange **221a** and compliant member (not shown) in the channel **362** within the control housing **361.** (In these figures, like numbered items correspond to similar components across different figures.)

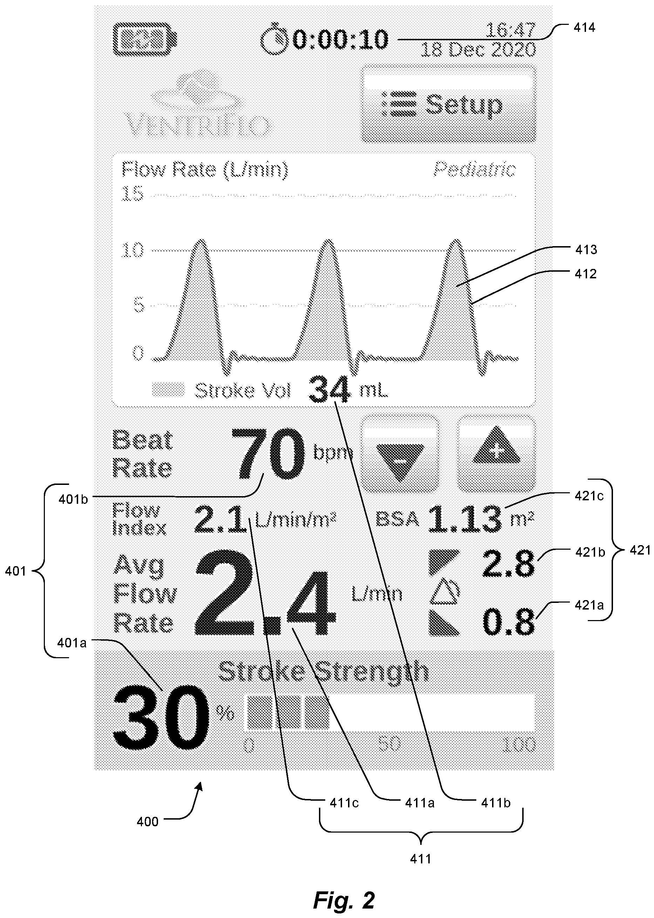

Fig. **2** is an example of a touch-sensitive graphic display **400** user interface showing user-specifiable motor parameters **401.** In this interface appear parameters stroke strength **401a** and beat rate **401b.** These parameters are a subset of user-specifiable parameters defining the performance of the linear motor. Additionally, in this interface appear flow characteristics **411** (average flow rate **411a**, stroke volume **411b,** flow index **411c),** flow rate waveform **412,** stroke volume waveform **413,** and timer **414.** These flow-based attributes are a subset of physical flow characteristics. Additionally, the user interface displays user-specifiable inputs **421** (low flow limit **421a**, high flow limit **421b,** and body surface area **421c).**

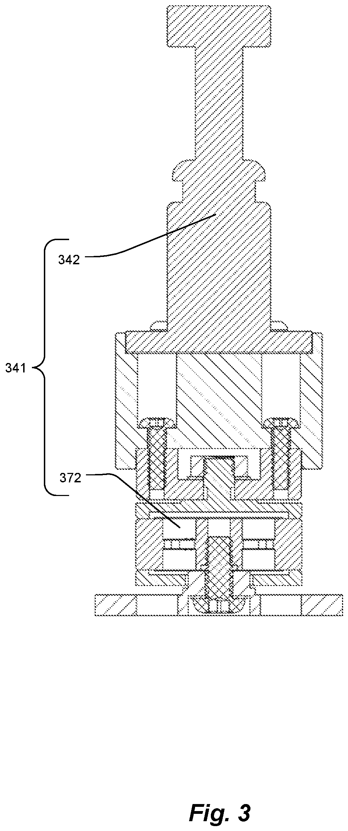

Fig. **3** is a vertical section of the push rod assembly **341** comprised of a push rod **342** and force sensor **372.**

Fig. **4** is a vertical section of the linear motor **330,** showing the coil **332** with electrical input **332a**, the frame **333,** the magnet centering spring **338,** and the magnet **339.** Other components and detail of the motor are provided in Fig. **1****.**

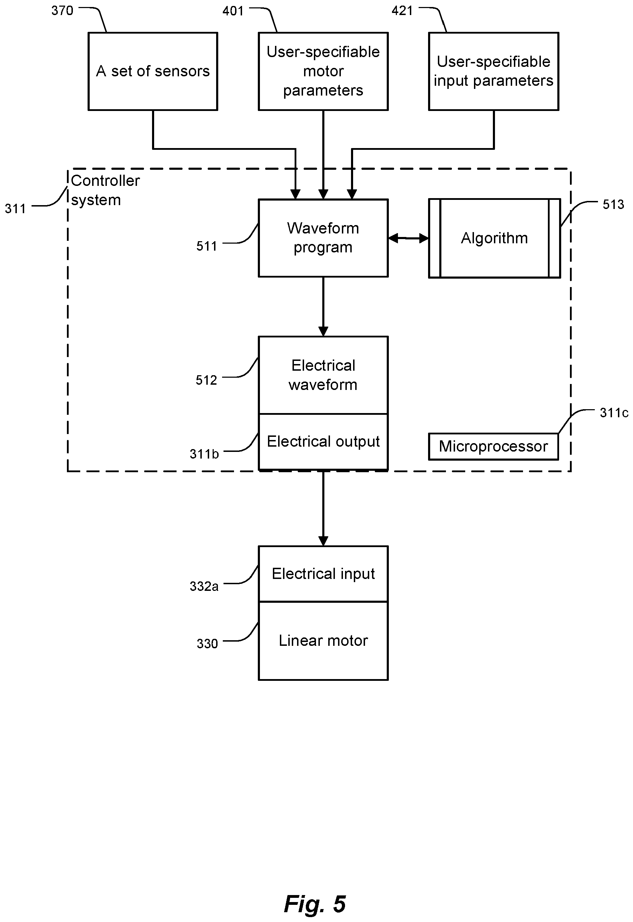

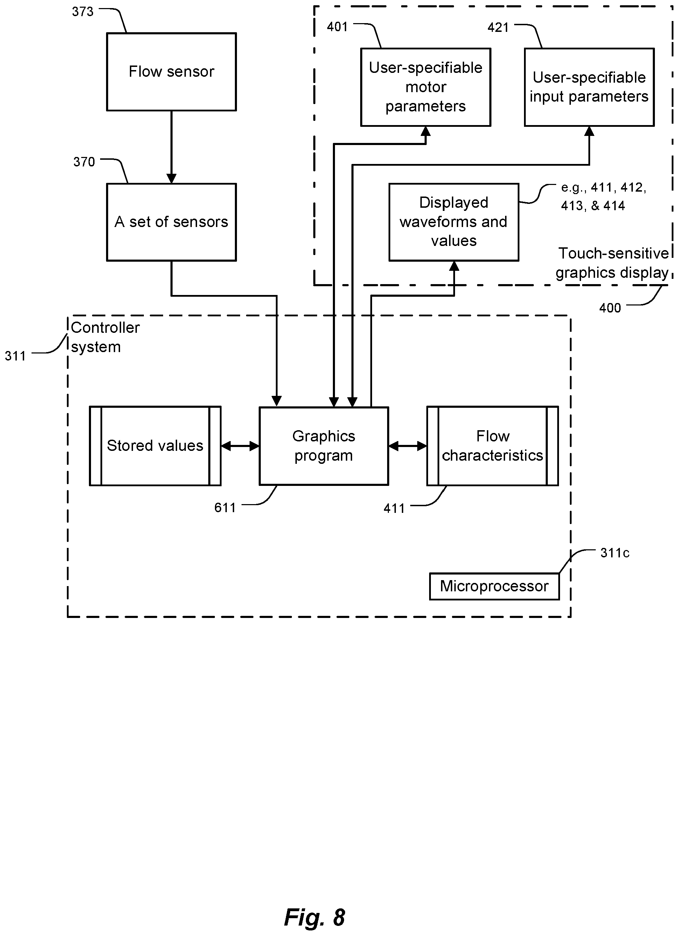

In Fig. **5****,** the waveform program **511** is a computer program executed by the controller system **311** microprocessor **311c** which accepts input from a set of sensors **370** (including position sensor **371** of Fig. **1****,** force sensor **372** of Figs. **1** and **3****,** and an external flow sensor **373** of Fig. **8****),** a set of user-specifiable motor parameters **401** (stroke strength **401a** and beat rate **401b)** defining performance of a linear motor **330** in the course of pumping. Additionally, Fig. **5** shows a set of user-specifiable input parameters **421** (low flow limit **421a**, high flow limit **421b,** and body surface area **421c).** The waveform program **511** outputs an electrical waveform **512,** the result of a set of algorithms **513,** at the electrical output **311b.** The electrical output **311b** is coupled to electrical input **332a** of linear motor **330.**

There is growing consensus that desirable characteristics of a pulsatile pump should include both sufficient hemodynamic energy and a human-like waveform architecture. To evaluate pulsatile flow, we choose the human heart as the best model: it delivers a proper stroke volume at a natural cadence with a physiologic rest at the end of each stroke, adapting to the physiologic demands of the patient by adjusting the cardiac output, as the product of stroke volume and beat rate. Via the left ventricle, the human heart provides hemodynamic energy that results in a pressure wave that propagates fully through the elastic arterial tree. It appears that only a biomimetic stroke volume delivered in a biomimetic time frame (like the native systolic contraction produced by the heart) allows the elastic arterial tree to properly relax during the diastolic phase. Use of continuous flow devices stretches the elastic arterial wall but never allows proper relaxation, creating constant and atypical stress on the endothelial cells and interfering with natural baroreceptor sympathetic and parasympathetic signaling, thus disrupting the body's homeostatic control state.

The waveform program **511** causes the pulsatile fluid pump system **301** to replicate the ability of the left ventricle of the human heart to deliver physiological hemodynamic energy proportional to a user-specified stroke strength **401a** by causing delivery of the necessary fraction of the stroke volume of a pump chamber **102** in a physiologic natural cadence at a user-specified beat rate **401b.** It is a user (a perfusionist) of the pulsatile fluid pump system **301** who adjusts the stroke strength **401a** (an indirect specification of stroke volume) and beat rate **401b** to meet the physiologic demand of the patient. Furthermore, the waveform program **511** replicates the physiologic rest at the end of each stroke, thereby allowing natural relaxation of the arterial tree.

The structure of a pulsatile pump in accordance with various embodiments of the present invention can usefully reflect attributes of the human heart. The human heart is preload sensitive-the heart cannot "pull" blood into the left ventricle; it can only allow the blood available to flow naturally into the ventricle. The human heart is also afterload sensitive in that it is responsive to the compliance and resistance in the downstream vasculature and doesn't exert excess force on the blood, which could damage the vasculature. Lastly, the left ventricle cannot deliver blood that isn't in the ventricle when it contracts; there is a limited bolus of blood that it can deliver.

The pulsatile fluid pump system **301** has similar attributes of inherent safety: it is preload and afterload sensitive, and it is limited in both the volume of blood it can deliver and the force at which it can deliver that bolus of blood. When filling, the pulsatile fluid pump system **301** allows gravity filling from the venous reservoir, exerting minimal negative pressure. When emptying, the linear motor **330** is inherently limited in the force that it can generate by its design. As such, it cannot overpressure the downstream tubing or vasculature, instead delivering less than the volume of blood in the pump chamber **102,** thereby only delivering as much volume as the vasculature can receive.

The integral pump assembly **200** is analogous to a left ventricle of the human heart; the inlet ball check valve assembly used in various embodiments hereof is analogous to a mitral valve; and the outlet ball check valve assembly used in various embodiments hereof is analogous to an aortic valve. Like the human heart, the inlet and outlet ball check valve assemblies are passive and require a slight reversal of flow to close. This slight reversal of flow mimics the slight reversal that occurs when the aortic valve of the human heart closes.

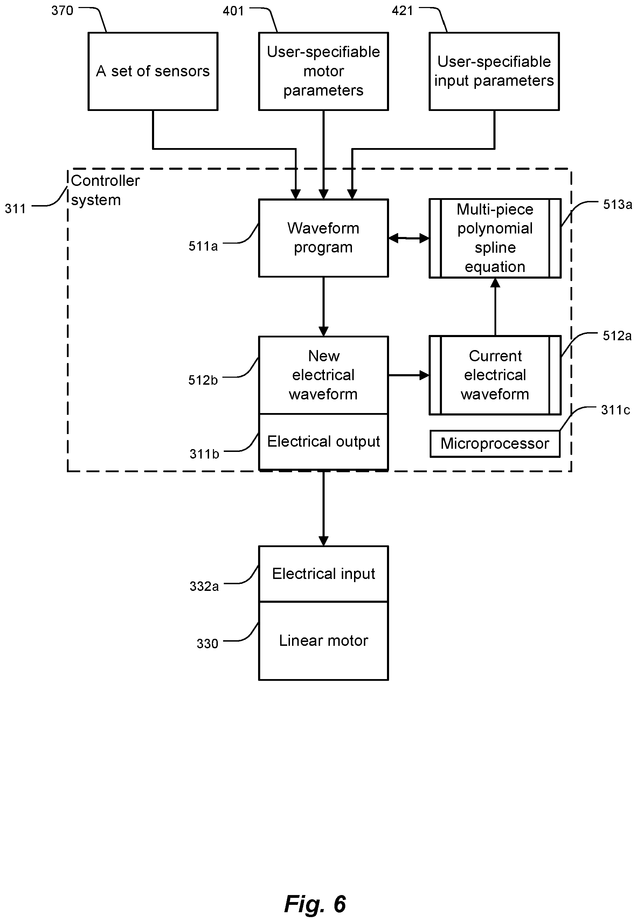

In one embodiment of the present invention, shown in Fig. **6****,** the waveform program **511a** is a computer program executed by the controller system **311** microprocessor **311c** which accepts input from a set of sensors **370,** a set of user-specifiable motor parameters **401,** and a set of user-specifiable input parameters **421.** The waveform program **511a** is configured to simulate a waveform that has been experimentally determined to be appropriate for embodiments of the pulsatile fluid pump system **301** of the present invention. The waveform program **511a** simulates the experimentally determined waveform by repeatedly performing a multi-piece polynomial spline algorithm **513a** and the resulting waveform is used to drive the linear motor **330.** In the event that the user changes one of the user-specifiable motor parameters **401,** the waveform program **511a** uses zero or more of the current and/or previous values from the set of sensors **370,** along with the set of user-specifiable motor parameters **401,** zero or more flow characteristics **411,** zero or more user-specifiable input parameters **421,** and the current electrical waveform **512a** to create a new electrical waveform **512b.** The waveform program **511a** outputs the new electrical waveform **512b,** consisting of discrete output voltages at defined time durations, at the electrical output **311b.** The electrical output **311b** is coupled to electrical input **332a** of linear motor **330.**

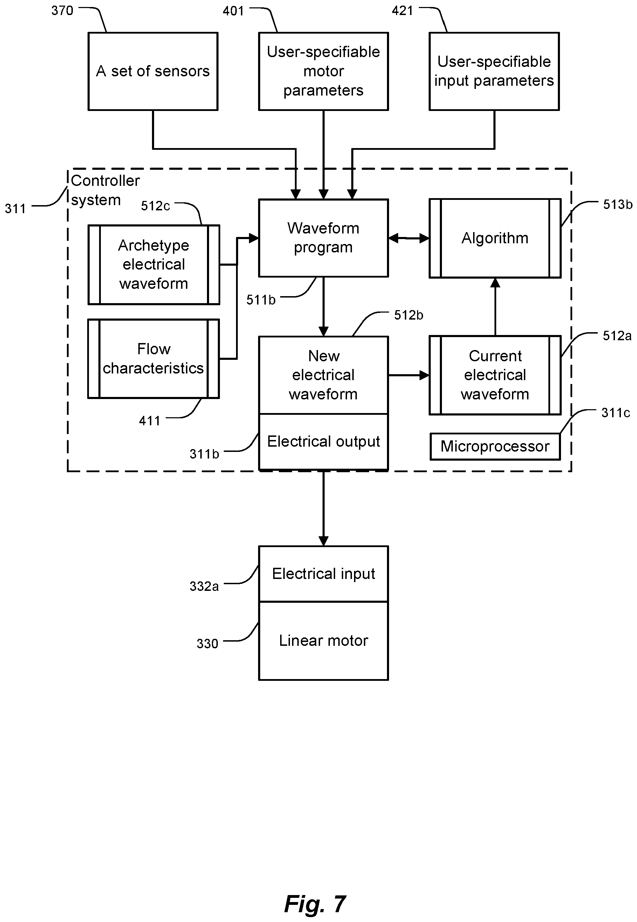

In another embodiment of the present invention, shown in Fig. **7****,** the waveform program **511b** is a computer program executed by the controller system **311** microprocessor **311c** which accepts input from a set of sensors **370,** a set of user-specifiable motor parameters **401,** and a set of user-specifiable input parameters **421.** The waveform program **511b** reads an archetype electrical waveform **512c** stored electronically within the controller system **311.** The waveform program **511b** then uses an algorithm **513b** to adjust the archetype electrical waveform **512c**. The algorithm **513b** creates a new electrical waveform **512b** from the archetype electrical waveform **512c** using zero or more of the current and/or previous values of the set of sensors **370,** along with the set of user-specifiable motor parameters **401,** zero or more flow characteristics **411,** zero or more user-specifiable input parameters **421,** and the current electrical waveform **512a.** The waveform program **511b** outputs the new electrical waveform **512b,** consisting of discrete output voltages at defined time durations, at the electrical output **311b.** The electrical output **311b** is coupled to electrical input **332a** of linear motor **330.**

In Fig. **8****,** the graphics program **611** is a computer program executed by the controller system **311,** which accepts user-specifiable motor parameters **401** and user-specifiable input parameters **421.** The graphics program **611** causes a set of current values of the user-specifiable motor parameters **401,** a set of flow characteristics **411,** and a set of user-specifiable input parameters **421** to be shown on the graphic display **400.**

When the stroke strength **401a** value transitions from zero to a positive value, the graphics program **611** sets timer **414** to zero, increments the timer **414** in real time, and causes, each second, the updated timer **414** value to be shown on the graphic display **400.** When the stroke strength **401a** value transitions from a positive value to zero, the graphics program **611** stops incrementing the timer **414** and causes the most recent value of timer **414** to be shown on the graphic display **400.**

The graphics program **611** accepts input from the flow sensor **373,** calculates the average flow rate **411a,** and causes the average flow rate **411a** to be shown on the graphic display **400.** The graphics program **611** also causes, in near real-time, the instantaneous flow rate as a flow rate waveform **412** to be shown on the graphic display **400.**

The graphics program **611** also uses data from the flow sensor **373** to calculate the average stroke volume **411b** and cause the average stroke volume **411b** to be shown on the graphic display **400.** The graphics program **611** also causes, in near real-time, the total volume of fluid currently delivered for a given stroke to be shown on the graphic display **400.** The total volume of fluid currently delivered for a given stroke is the integral of instantaneous flow as a stroke volume waveform **413** and is displayed as the shaded area under the flow rate waveform **412.**

The graphics program **611** accepts input of body surface area **421c** and calculates flow index **411c** as the average flow rate **411a** divided by the body surface area **421c.** The graphics program **611** further causes the body surface area **421c** and calculated flow index **411c** to be shown on the graphic display **400.**

The graphics program **611** accepts input of low flow limit **421a** and high flow limit **421b,** and causes the low flow limit **421a** and high **flow limit 421b** settings to be shown on the graphic display **400.**

The embodiments of the invention **described above are** intended to be merely exemplary. The scope of the present invention is defined by the appended claims.

## Claims

1. A pulsatile fluid pump system for driving a fluid pump assembly, the pulsatile fluid pump system comprising:
a reciprocating linear motor (330) having a magnet (339) and a coil (332), the magnet moving in relation to the coil, the coil having an electrical input (332a);
a controller system (311) having an electrical output (311b) coupled to the electrical input of the coil and a storage system in which is stored an archetype waveform (512c), the controller system being configured to execute a waveform program (511b) defining an electrical waveform (512b) at the electrical output;
wherein the waveform program is configured to accept user-provided values of a set of user-specifiable parameters defining performance of the linear motor and further configured to read the archetype electrical waveform from the storage system and to generate the electrical waveform at the electrical output by modifying the archetype electrical waveform with respect to a feature, selected from the group consisting of amplitude, frequency, shape, and combinations thereof, in response to the user-provided values for such parameters.

2. A pulsatile fluid pump system according to claim 1, further comprising a graphic display (400), coupled to the controller system (311), the controller system executing a graphics program (611) configured to cause the graphic display to show a set of user-specifiable parameters defining the performance of the linear motor.

3. A pulsatile fluid pump system according to claim 2, further comprising a flow sensor (373) mechanically coupled to a fluid path including the integrated pump assembly, the flow sensor having an electrical output coupled to the controller system, wherein the controller system is executing a graphics program (611) configured to cause the graphic display (400) to show a set of items, including values of a set of user-specifiable parameters defining the performance of the linear motor and values of a set of physical flow characteristics.

4. A pulsatile fluid pump system according to claim 3, wherein the set of items shown includes an instantaneous flow rate waveform in near real-time.

5. A pulsatile fluid pump system according to claim 3, wherein the set of items shown includes an instantaneous stroke volume waveform in near real-time.

6. A pulsatile fluid pump system according to claim 1, wherein the waveform program (511b) is configured to generate the electrical waveform (512b) at the electrical output (311b) by repeatedly performing a multi-piece polynomial spline algorithm in a manner responsive to a set of user-specifiable parameters defining the performance of the linear motor (330).

7. A pulsatile fluid pump system according to claim 1, further comprising a set of sensors (370), electrically coupled to the controller system (311) and configured to produce a set of sensor outputs corresponding to pumping performance, wherein the waveform program (511b) is configured to generate the electrical waveform (512b) at the electrical output in a manner responsive to the set of sensor outputs and a set of user-specifiable parameters.

8. A pulsatile fluid pump system according to claim 1, wherein the user of the pulsatile fluid pump system may choose from a set of waveform programs.

9. A pulsatile fluid pump system according to claim 2, wherein the graphic display (400) is touch sensitive.

## Patentansprüche

1. Pulsierendes Fluidpumpensystem zum Antreiben einer Fluidpumpenbaugruppe, das pulsierende Fluidpumpensystem umfassend:
einen hin- und hergehenden Linearmotor (330), der einen Magneten (339) und eine Spule (332) aufweist, wobei sich der Magnet relativ zu der Spule bewegt und die Spule eine elektrische Eingabe (332a) aufweist;
ein Steuerungssystem (311), das eine elektrische Ausgabe (311b), die mit der elektrischen Eingabe der Spule gekoppelt ist, und ein Speichersystem aufweist, in dem eine Archetypwellenform (512c) gespeichert ist, wobei das Steuerungssystem konfiguriert ist, ein Wellenformprogramm (511b) auszuführen, das eine elektrische Wellenform (512b) an der elektrischen Ausgabe definiert;
wobei das Wellenformprogramm konfiguriert ist, vom Benutzer bereitgestellte Werte eines Satzes von benutzerspezifizierbaren Parametern zu akzeptieren, die die Leistung des Linearmotors definieren, und ferner konfiguriert ist, die elektrische Archetypwellenform aus dem Speichersystem zu lesen und die elektrische Wellenform an der elektrischen Ausgabe durch Verändern der elektrischen Archetypwellenform in Bezug auf ein Merkmal zu erzeugen, das aus der Gruppe ausgewählt ist, bestehend aus Amplitude, Frequenz, Form und Kombinationen davon, als Antwort auf die vom Benutzer bereitgestellten Werte für solche Parameter.

2. Pulsierendes Fluidpumpensystem nach Anspruch 1, ferner umfassend eine grafische Anzeige (400), die mit dem Steuerungssystem (311) gekoppelt ist, wobei das Steuerungssystem ein Grafikprogramm (611) ausführt, das konfiguriert ist, die grafische Anzeige zu veranlassen, einen Satz von benutzerspezifizierbaren Parametern anzuzeigen, die die Leistung des Linearmotors definieren.

3. Pulsierendes Fluidpumpensystem nach Anspruch 2, ferner umfassend einen Flusssensor (373), der mechanisch mit einem Fluidpfad gekoppelt ist, einschließend die integrierte Pumpenbaugruppe, wobei der Flusssensor eine elektrische Ausgabe hat, der mit dem Steuerungssystem gekoppelt ist, wobei das Steuerungssystem ein Grafikprogramm (611) ausführt, das konfiguriert ist, die grafische Anzeige (400) zu veranlassen, einen Satz von Elementen anzuzeigen, einschließend Werte eines Satzes von benutzerspezifizierbaren Parametern, die die Leistung des Linearmotors definieren, und Werte eines Satzes von physikalischen Flusseigenschaften.

4. Pulsierendes Fluidpumpensystem nach Anspruch 3, wobei der gezeigte Satz von Elementen eine momentane Strömungsratenwellenform in nahezu Echtzeit einschließt.

5. Pulsierendes Fluidpumpensystem nach Anspruch 3, wobei der gezeigte Satz von Elementen eine momentane Hubvolumenwellenform in nahezu Echtzeit einschließt.

6. Pulsierendes Fluidpumpensystem nach Anspruch 1, wobei das Wellenformprogramm (511b) konfiguriert ist, die elektrische Wellenform (512b) an der elektrischen Ausgabe (311b) durch wiederholtes Durchführen eines mehrteiligen Polynom-Spline-Algorithmus in einer Weise zu erzeugen, die auf einen Satz von benutzerspezifizierbaren Parametern reagiert, die die Leistung des Linearmotors (330) definieren.

7. Pulsierendes Fluidpumpensystem nach Anspruch 1, ferner umfassend einen Satz von Sensoren (370), die elektrisch mit dem Steuerungssystem (311) gekoppelt und konfiguriert sind, einen Satz von Sensorausgaben entsprechend der Pumpleistung zu erzeugen, wobei das Wellenformprogramm (511b) konfiguriert ist, die elektrische Wellenform (512b) an der elektrischen Ausgabe in einer Weise zu erzeugen, die auf den Satz von Sensorausgaben und einen Satz von benutzerspezifizierbaren Parametern reagiert.

8. Pulsierendes Fluidpumpensystem nach Anspruch 1, wobei der Benutzer des pulsierenden Fluidpumpensystems aus einem Satz von Wellenformprogrammen wählen kann.

9. Pulsierendes Fluidpumpensystem nach Anspruch 2, wobei die grafische Anzeige (400) berührungsempfindlich ist.

## Revendications

1. Système de pompe à fluide pulsatile pour entraîner un ensemble pompe à fluide, le système de pompe à fluide pulsatile comprenant :
un moteur linéaire alternatif (330) ayant un aimant (339) et une bobine (332), l'aimant se déplaçant par rapport à la bobine, la bobine ayant une entrée électrique (332a) ;
un système de commande (311) ayant une sortie électrique (311b) couplée à l'entrée électrique de la bobine et un système de stockage dans lequel est stockée une forme d'onde archétypique (512c), le système de commande étant configuré pour exécuter un programme de forme d'onde (511b) définissant une forme d'onde électrique (512b) au niveau de la sortie électrique ;
dans lequel le programme de forme d'onde est configuré pour accepter des valeurs fournies par l'utilisateur d'un ensemble de paramètres pouvant être spécifiés par l'utilisateur définissant des performances du moteur linéaire et configuré en outre pour lire la forme d'onde électrique archétypique provenant du système de stockage et pour générer la forme d'onde électrique au niveau de la sortie électrique en modifiant la forme d'onde électrique archétypique par rapport à une caractéristique, sélectionnée dans le groupe constitué d'une amplitude, d'une fréquence, d'une forme et de combinaisons de celles-ci, en réponse aux valeurs fournies par l'utilisateur pour de tels paramètres.

2. Système de pompe à fluide pulsatile selon la revendication 1, comprenant en outre un dispositif d'affichage graphique (400), couplé au système de commande (311), le système de commande exécutant un programme graphique (611) configuré pour amener le dispositif d'affichage graphique à présenter un ensemble de paramètres pouvant être spécifiés par l'utilisateur définissant les performances du moteur linéaire.

3. Système de pompe à fluide pulsatile selon la revendication 2, comprenant en outre un capteur d'écoulement (373) couplé mécaniquement à un trajet de fluide comportant l'ensemble pompe intégré, le capteur d'écoulement ayant une sortie électrique couplée au système de commande, dans lequel le système de commande exécute un programme graphique (611) configuré pour amener le dispositif d'affichage graphique (400) à présenter un ensemble d'éléments, comportant des valeurs d'un ensemble de paramètres pouvant être spécifiés par l'utilisateur définissant les performances du moteur linéaire et des valeurs d'un ensemble de caractéristiques d'écoulement physique.

4. Système de pompe à fluide pulsatile selon la revendication 3, dans lequel l'ensemble d'éléments présentés comporte une forme d'onde de débit instantanée en temps quasi réel.

5. Système de pompe à fluide pulsatile selon la revendication 3, dans lequel l'ensemble d'éléments présentés comporte une forme d'onde de volume systolique instantanée en temps quasi réel.

6. Système de pompe à fluide pulsatile selon la revendication 1, dans lequel le programme de forme d'onde (511b) est configuré pour générer la forme d'onde électrique (512b) au niveau de la sortie électrique (311b) en réalisant de manière répétée un algorithme de splines polynomiales en plusieurs morceaux de manière à répondre à un ensemble de paramètres pouvant être spécifiés par l'utilisateur définissant les performances du moteur linéaire (330).

7. Système de pompe à fluide pulsatile selon la revendication 1, comprenant en outre un ensemble de capteurs (370), couplés électriquement au système de commande (311) et configurés pour produire un ensemble de sorties de capteur correspondant à des performances de pompage, dans lequel le programme de forme d'onde (511b) est configuré pour générer la forme d'onde électrique (512b) au niveau de la sortie électrique de manière à répondre à l'ensemble de sorties de capteur et à un ensemble de paramètres pouvant être spécifiés par l'utilisateur.

8. Système de pompe à fluide pulsatile selon la revendication 1, dans lequel l'utilisateur du système de pompe à fluide pulsatile peut choisir parmi un ensemble de programmes de forme d'onde.

9. Système de pompe à fluide pulsatile selon la revendication 2, dans lequel le dispositif d'affichage graphique (400) est tactile.
